(19) Europäisches Patentamt
European Patent Office
Office européen des brevets

(11) EP 1 140 807 B1

(12) **FASCICULE DE BREVET EUROPEEN**

(45) Date de publication et mention de la délivrance du brevet:
**25.02.2004 Bulletin 2004/09**

(21) Numéro de dépôt: **99961146.0**

(22) Date de dépôt: **22.12.1999**

(51) Int Cl.[7]: **C07C 291/04**, C07D 295/22, C07D 211/94, C07D 207/46, C07B 61/02, C07F 9/40

(86) Numéro de dépôt international:
**PCT/FR1999/003254**

(87) Numéro de publication internationale:
**WO 2000/040550 (13.07.2000 Gazette 2000/28)**

(54) **PROCEDE DE PREPARATION DE NITROXYDES**

VERFAHREN ZUR HERSTELLUNG VON NITROXIDEN

METHOD FOR PREPARING NITROXIDES

(84) Etats contractants désignés:
**AT BE CH CY DE DK ES FI FR GB GR IE IT LI LU MC NL PT SE**

(30) Priorité: **08.01.1999 FR 9900128**

(43) Date de publication de la demande:
**10.10.2001 Bulletin 2001/41**

(73) Titulaire: **Atofina**
**92800 Puteaux (FR)**

(72) Inventeurs:
- **GILLET, Jean-Philippe**
**F-69530 Brignais (FR)**
- **GUERRET, Olivier**
**F-69280 Marcy l'Etoile (FR)**
- **LASCOMBE, Jean-Pierre**
**F-69350 La Mulatiere (FR)**

(56) Documents cités:
**WO-A-96/24620**
**FR-A- 2 036 557**
**US-A- 4 845 090**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 140 807 B1

## Description

La présente invention concerne un procédé de préparation de radicaux N-oxyde d'amines secondaires par oxydation des amines secondaires correspondantes.

Les radicaux N-oxyde d'amines secondaires, désignés ci-après par nitroxydes sont des radicaux libres contenant un électron célibataire. Ces radicaux nitroxydes peuvent être utilisés comme agents stabilisants des polymères organiques, notamment pour inhiber la dégradation thermique et par la lumière des polyoléfines (US 3,431,233) et du PVC (US 3,547,874). Ils sont également utilisés comme marqueur de spin pour l'étude des composés biologiques et comme régulateurs des polymérisations radicalaires (Georges, M, K et col. Macromoleculules 1993, 26, 2987).

Les principales méthodes de préparation des nitroxydes sont l'oxydation des hydroxylamines et l'oxydation d'amines secondaires, cette seconde méthode étant la plus fréquemment utilisée industriellement.

Les méthodes d'obtention des radicaux nitroxydes par oxydation des amines secondaires correspondantes ont été largement décrites.

Parmi ces méthodes, on citera celles qui utilisent l'eau oxygénée en présence de catalyseurs tels que l'acide phosphotungstique (Brière R. et coll., Bulletin de la Société Chimique de France, 1965, pages 3273-3282), les sels des métaux alcalino-terreux (EP 0 574 607), $NaHCO_3$ ou $Na_2CO_3$ (LEVINA et coll. Dokl. Akad. Nauk. SSSR 1981, 261(1), 109-110), l'EDTA associée au $WO_4Na_2$ (Zakizewski J., J. Prakt. Chem. 1985, 327(6), 1011-1014).

Dans toutes ces méthodes, la réaction d'oxydation a lieu dans l'eau ou un mélange eau/alcool à des températures comprises entre 60 et 100°C.

Bien qu'utilisant un oxydant peu onéreux - $H_2O_2$ - ces méthodes ne sont applicables qu'à des couples amine/nitroxyde suffisamment solubles dans l'eau. Elles présentent également des désavantages qui résident notamment dans des durées de réaction longues et dans l'impossibilité de recycler le système catalytique ce qui nécessite de laver les produits et de traiter les effluents avant leur élimination.

En outre, ces méthodes ne sont pas générales car les températures utillisées, généralement élevées, rendent difficile voire impossible l'isolation de certains nitroxydes facilement suroxydables.

D'autres méthodes d'oxydation des amines secondaires en nitroxydes ont été proposées.

Ainsi, dans le brevet US 4,665,185, on décrit un procédé d'oxydation d'amine secondaire cyclique qui consiste à faire réagir ladite amine dans un solvant organique inerte, avec un hydroperoxyde organique tel que l'hydroperoxyde de tert-butyle, en présence de quantités catalytiques d'un métal carbonyle ($Mo(CO)_6$), d'un oxyde de métal ($MoO_3$), d'un acétylacétonate de métal ou d'un alcoolate de métal ($Ti(OiPr)_4$) dont le métal appartient à l'un des groupes IVB, VB, VIB, VIIB et VIII de la classification périodique, à une température allant de 0°C à 200°C.

Les durées de réaction sont plus courtes mais le problème de la récupération du catalyseur subsiste toujours. Les produits doivent être purifiés et les effluents traités.

Une autre méthode d'oxydation d'amine secondaire consiste à faire réagir ladite amine secondaire avec le diméthyldioxirane (DMD) en absence de catalyseurs (US 5,087,752).

Le DMD, non commercialisé, est préparé par oxydation de l'acétone soit avec l'OXONE® qui est un monoperoxysulfate de potassium, soit plus rarement avec l'acide de Caro qui est un mélange $H_2SO_4/H_2SO_5$.

Cet oxydant puissant permet l'obtention de rendements élevés en nitroxydes. Cependant, son fort caractère explosif rend très aléatoire sa préparation et est rédhibitoire pour une utilisation industrielle.

De plus, il est nécessaire pour obtenir le DMD d'employer l'OXONE® en excès en milieu basique, ce qui engendre une grande quantité de sulfate comme effluents.

Les acides perbenzoiques tels que l'acide métachloroperbenzoique (Journal of American Chemical Society, 1967, 89(12) 3055-3056) ou l'acide paranitroperbenzoique (Rassat A. et coll. Bulletin de la Société Chimique de France, 1965, 3283-3290) en solution dans des solvants organiques tels que le chlorure de méthylène permettent d'oxyder - efficacement de nombreuses amines secondaires en nitroxydes.

. Cependant, leur coût et les acides benzoïques qu'ils engendrent rendent inexploitables leur utilisation à l'échelle industrielle.

Le brevet US 4,845,090 utilise l'acide peracétique en présence d'acétate de sodium et en solution dans des solvants organiques ($CH_2CP_2$).

On a maintenant trouvé que l'on pouvait obtenir des nitroxydes. selon un procédé qui évite les inconvénients précités, qui consiste à oxyder des amines secondaires au moyen de peracides aliphatiques en milieu biphasique solvant organique / eau dont la phase aqueuse est maintenue à un pH allant de 4 à 12.

La présente invention a donc pour objet un procédé de préparation de radicaux N-oxyde d'amines secondaires (radicaux nitroxydes) à partir des amines secondaires correspondantes, caractérisé en ce que l'on effectue les étapes suivantes :

a/ on solubilise l'amine secondaire avec un solvant organique, non miscible à l'eau, puis on ajoute de l'eau,

b/ on ajoute ensuite simultanément et sous forte agitation au milieu biphasique ainsi obtenu, une quantité de

peracide aliphatique selon un rapport molaire peracide / amine secondaire allant de 1,5 à 2,5 et, de préférence, allant de 1,5 à 2, et une quantité suffisante d'une solution aqueuse basique pour obtenir un pH de la phase aqueuse du milieu biphasique allant de 4 à 12, à une température comprise entre -10°C et +40°C, jusqu'à conversion complète de l'amine secondaire, puis

c/ on récupère la phase organique par simple décantation, et on isole le nitroxyde par évaporation du solvant organique sous pression réduite.

Selon la présente invention, le solvant organique doit être non miscible à l'eau, inerte vis-à-vis des réactifs et des produits et avoir un bon pouvoir solvant des réactifs et des produits obtenus.

A titre d'illustration de tels solvants organiques utilisables selon la présente invention, on citera les hydrocarbures aliphatiques ou cycloaliphatiques tels que le pentane, l'hexane, l'heptane, le décane, le cyclohexane, le cyclododécane ; les hydrocarbutes aromatiques tels que le benzène, le toluène, les xylènes; les hydrocarbures chlorés tels que le chlorure de méthylène, le 1,2-dichloroéthane, le chloroforme, le chlorobenzène ; les esters d'acides aliphatiques tels que l'acétate d'éthyle, le propionate d'éthyle ou un mélange d'au moins deux des solvants précités.

A titre d'illustration de peracides aliphatiques utilisables selon la présente invention, on citera l'acide peracétique, l'acide perpropionique, l'acide perbutanoïque.

De préférence, on utilisera l'acide peracétique ou l'acide perpropionique.

Le procédé de la présente invention consiste donc à solubiliser, sous agitation, l'amine secondaire dans un solvant organique, non miscible à l'eau et inerte vis-à-vis de ladite amine secondaire et des réactifs mis en oeuvre.

La quantité de solvant organique utilisée est fonction de la solubilité de ladite amine secondaire dans ledit solvant.

Cette quantité de solvant organique n'est pas critique et peut varier dans une large mesure. Cependant, pour des motifs économiques et dans un souci de respect de l'environnement, l'homme du métier s'efforcera de choisir un solvant organique (ou un mélange de solvants) de façon à n'utiliser que des quantités minimales pour solubiliser l'amine secondaire.

A la solution organique, on ajoute; sous agitation, de l'eau, puis on ajoute simultanément sous forte agitation, au milieu biphasique obtenu le peracide aliphatique, généralement en solution dans l'acide aliphatique correspondant, selon un rapport molaire peracide/amine secondaire tel que défini précédemment et une quantité suffisante d'une solution aqueuse basique d'un carbonate ou d'un hydrogénocarbonate d'un métal alcalin ou d'un métal alcalino-terreux de manière à ce que le pH de la solution aqueuse du milieu biphasique soit maintenu à une valeur allant de 4 à 12 et, de préférence, allant de 5 à 9.

Selon la présente invention, on utilisera, de préférence, une solution aqueuse basique d'un carbonate ou d'un hydrogénocarbonate d'un métal alcalin.

A titre d'illustration de carbonates ou d'hydrogénocarbonates de métaux alcalins utilisables selon la présente invention, on citera $NaHCO_3$, $KHCO_3$, $K_2CO_3$, $Na_2CO_3$.

On peut également utiliser des solutions ammoniacales.

La concentration pondérale des solutions aqueuses en carbonates ou en hydrogénocarbonates des métaux alcalins ou alcalino-terreux est fixée par la limite de solubilité de ces espèces dans l'eau. On s'efforcera d'utiliser des solutions les plus concentrées possibles.

La réaction d'oxydation de l'amine secondaire en nitroxyde selon le schéma :

$$> N — H + {}^{3}/_{2}\, RCO_3H \rightarrow\, > N — O^{\bullet} + {}^{3}/_{2}\, RCO_2H + {}^{1}/_{2} H_2O$$

est exécutée à une température allant de -10°C à +40°C et, de préférence, allant de -5°C à + 30°C jusqu'à ce que l'amine secondaire soit totalement transformée. L'avancement de la réaction peut être suivi par des méthodes analytiques telles que le dosage chimique du peracide mis en oeuvre selon des méthodes connues de l'homme du métier (dosage au sulfite et au $Ce^{4+}$) et la chromatographie en phase gazeuse.

On opère de préférence à pression atmosphérique. La réaction terminée, on récupère la phase organique, généralement, par simple décantation. Cette phase organique peut être éventuellement lavée avec une eau déminéralisée puis séchée.

On isole le nitroxyde formé par évaporation du solvant organique sous pression réduite. Si nécessaire, la pureté du nitroxyde peut être améliorée par distillation sous pression réduite ou par recristallisation. Les nitroxydes peuvent être identifiés par analyse élémentaire, HPLC, IR, RPE et spectrométrie de masse (SM).

Selon le procédé de l'invention, la réaction d'oxydation des amines secondaires en nitroxydes présente l'avantage d'être effectuée avec des oxydants peu coûteux, commercialement disponibles, selon des conditions douces, rapidement et avec des rendements élevés.

En outre, la purification des nitroxydes obtenus est simple et les effluents (sels d'acides organiques) peuvent

être éventuellement valorisés.

Le procédé d'oxydation des amines secondaires en nitroxydes selon l'invention présente l'avantage de pouvoir s'appliquer aux amines secondaires (ou polyamines secondaires) à empêchement stérique ou à celles possédant un —CH en $\alpha$ de l'atome d'azote.

Par amines secondaires à empêchement stérique, on désigne présentement les amines qui comportent au moins un motif (A) tel que décrit ci-après :

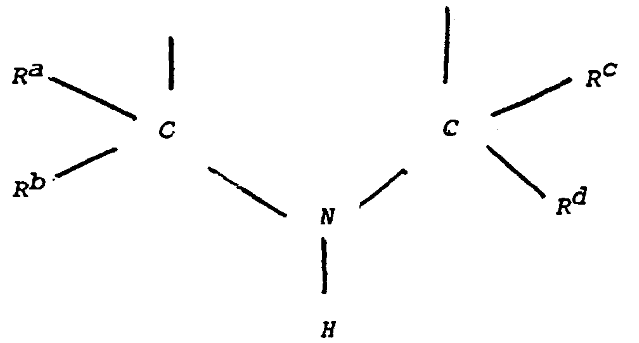

(A)

dans lequel $R^a$, $R^b$, $R^c$ et $R^d$, identiques ou différents, représentent un radical alkyle, linéaire ou ramifié, ayant un nombre d'atomes de carbone allant de 1 à 10, ou $R^a$ et $R^b$ forment avec l'atome de carbone qui les porte, ou $R^c$ et $R^d$ forment avec l'atome de carbone qui les porte un cycloalkyle ayant un nombre d'atomes de carbone allant de 3 à 10, un reste spiranique, cholestanique ou androstanique.

Aussi, le procédé d'oxydation de la présente invention permet d'oxyder en nitroxyde les amines secondaires à empêchement stériquë comprenant au moins 1 motif (A) ou les amines secondaires possédant au moins 1—CH en $\alpha$ de l'atome d'azote choisies parmi les composés représentés par les formules suivantes, dans lesquelles $R^a$, $R^b$, $R^c$ et $R^d$ ont les significations précédemment mentionnées :

- les composés de formule (I) :

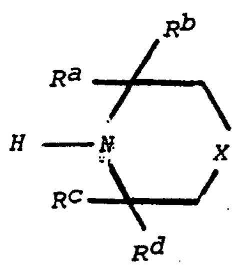

(I)

dans laquelle X représente un radical divalent choisi parmi les radicaux suivants: $>CH_2$ ; $>C{=}O$ ; —O— ;

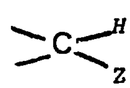

dans lequel Z représente un reste monovalent choisi parmi —CN, —NHR, —OR, —N=C(R)$_2$ dans lesquels R représente un atome d'hydrogène, un radical alkyle, linéaire ou ramifié ayant un nombre d'atomes de carbone allant de 1 à 10, un radical benzyle, un radical phényle, —CN, —C(O)$R^1$ ; C=N—$R^1$, C(O$R^1$)$_2$ ou $R^1$ a la même signification que R ;

- les composés de formule (II) :

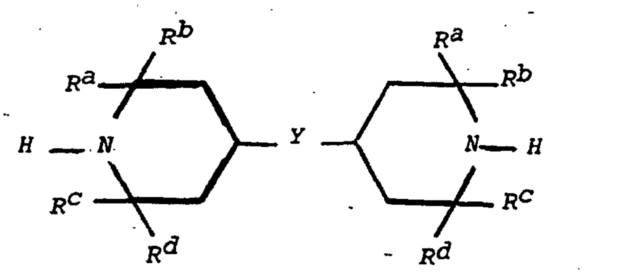

(II)

dans laquelle Y représente un reste divalent choisi parmi :

—OC(O)—$(CR^2R^3)_n$—C(O)O—,

—NH—$(CR^2R^3)_n$NH—,

—NHC(O)—$(CR^2R^3)_n$—C(O)NH,

—S—, —O—, $R^2$ et $R^3$, identiques ou différents, représentent un atome d'hydrogène, un radical alkyle, linéaire ou ramifié, ayant un nombre d'atomes de carbone allant de 1 à 10, n représentent un nombre entier allant de 0 à 20 ;

- les composés de formule (III) :

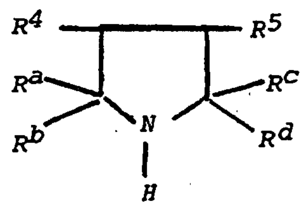

(III)

dans laquelle. $R^4$ représente un atome d'hydrogène un radical alkyle, linéaire ou ramifié, ayant un nombre d'atomes de carbone allant de 1 à 10, $R^5$ représenté un atome d'hydrogène —$CH_3$, —$C_2H_5$, —$CH_2OH$, —$C(O)NH_2$, —OH, —CHO ;

- les composés de formule (IV) :

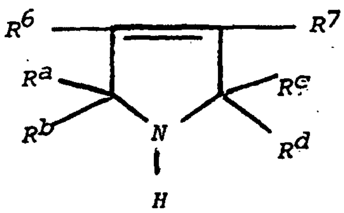

(IV)

dans laquelle $R^6$ a la même signification que $R^4$ et $R^7$ a la même signification que $R^5$,

- les 1,1,3,3-tétraméthyl-pyrrolopyridines de formule (V) :

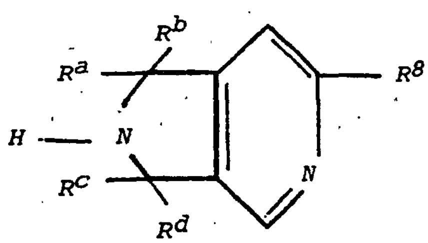

(V)

et les 1,1,3,3-tétraméthyl-2,3-dihydroisoindoles de formule (VI) :

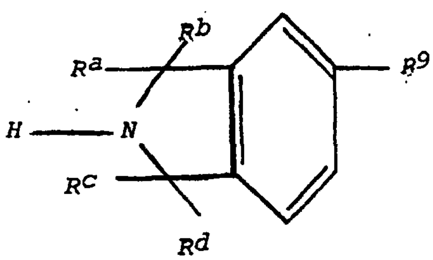

(VI)

dans lesquelles $R^8$ et $R^9$ représentent un atome d'hydrogène, un radical alkyle, linéaire ou ramifié, ayant un nombre d'atomes de carbone allant de 1 à 10 ;

- les composés de formule (VII) :

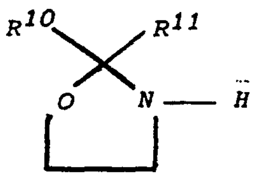

(VII)

dans laquelle $R^{10}$ et $R^{11}$, identiques ou différents, représentent un radical alkyle, linéaire ou ramifié, ayant un nombre d'atomes de carbone allant de 1 à 20, un radical carboxyalkyle $-(CH_2)mCO_2H$ dans lequel m = 1 à 20 ;

- les composés de formule (VIII) :

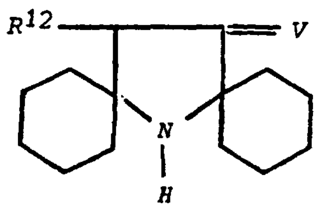

(VIII)

dans laquelle $R^{12}$ représente un atome d'hydrogène, un reste alkyle, linéaire ou ramifié ayant un nombre d'atomes de carbone allant de 1 à 20, un radical —C(O)$NH_2$, V =0 ; S, NH;

- les composés de formule (IX) :

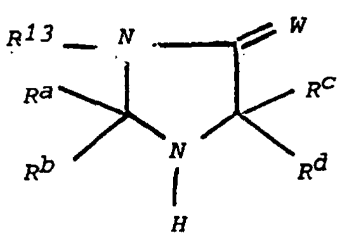

(IX)

dans laquelle $R^{13}$ a la même signification que $R^{12}$ et W a la même signification que V de la formule (VIII) ;

- les composés de formule (X) :

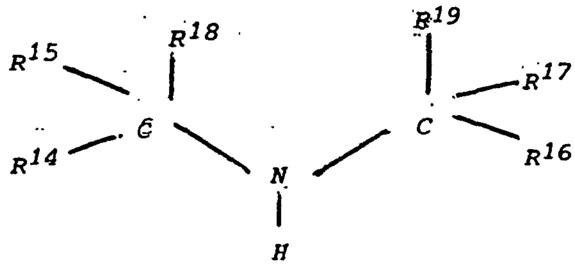

(X)

dans lesquels $R^{14}$, $R^{15}$, $R^{16}$, $R^{17}$, $R^{18}$, et $R^{19}$, identiques ou différents représentent un radical alkyle, linéaire ou ramifié, ayant un nombre d'atomes de carbone allant de 1 à 20, un radical cycloalkyle ayant au moins 3 atomes de carbone; un radical phényle, un radical benzyle, ou bien $R^{14}$ et $R^{15}$ forment avec l'atome de carbone qui les porte, ou bien $R^{16}$ et $R^{17}$ forment avec l'atome de carbone qui les porte, un cycloalkyle ayant un nombre d'atomes de carbone allant de 3 à 10 ;

- la 1,2,3,6-tétrahydro-2,2,6,6-tétraméthylpyridine :
- la 3,3-diméthyl-1-oxa-4-azaspiro[4.5]décane :
- la 2,3,3,5,5-pentaméthylmorpholine :
- la 3,3,5,5-tétraméthyl-2-méthylènemorpholine,
- le N-(2,2,6,6-tétraméthylpipéridyl-4)-ε-caprolactame :
- le 4,4'diméthylspiro(5α-cholestane-3,2'oxazolidine) :
- les composés de formule (XI) :

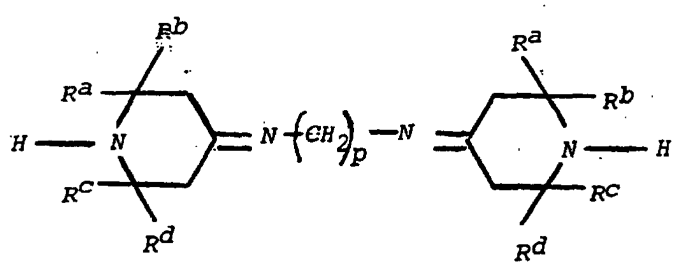

(XI)

avec p = 0 à 20.

- les composés de formule (XII) :

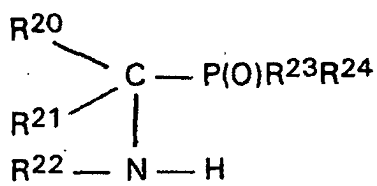

(XII)

dans laquelle $R^{20}$ et $R^{21}$, identiques ou différents, représentent un atome d'hydrogène, un radical alkyle linéaire, ramifié ou cyclique ayant un nombre d'atomes de carbone allant de 1 à 10, un radical aryle, un radical aralkyle, ou bien $R^{20}$ et $R^{21}$ sont reliés entre eux de façon à former un cycle incluant l'atome de carbone portant lesdits radicaux $R^{20}$ et $R^{21}$, ledit cycle ayant un nombre d'atomes de carbone, incluant le carbone porteur des radicaux $R^{20}$ et $R^{21}$, allant de 3 à 8 ; $R^{22}$ représente un radical hydrocarboné, linéaire ou ramifié, saturé ou insaturé, pouvant comprendre au moins un cycle, ledit radical ayant un nombre d'atomes de carbone allant de 1 à 30 ; $R^{23}$ et $R^{24}$, identiques ou différents, représentent un radical alkyle, linéaire ou ramifié, ayant un nombre d'atomes de carbone allant de 1 à 20, un radical cycloalkyle, aryle, alkoxyle, aryloxyle, aralkyloxyle, perfluoroalkyle, aralkyle, dialkyle ou diarylamino, alkylarylamino, thioalkyle, ou bien $R^{23}$ et $R^{24}$ peuvent également être reliés entre eux de façon à former un cycle incluant l'atome de phosphore, ledit hétérocycle pouvant avoir un nombre d'atomes de carbone

allant de 2 à 4 et pouvant contenir en outre 1 ou plusieurs atomes d'oxygène, de soufre ou d'azote ;

- les oxa-1-diaza-oxo-spirodécanes de formule (XIII) :

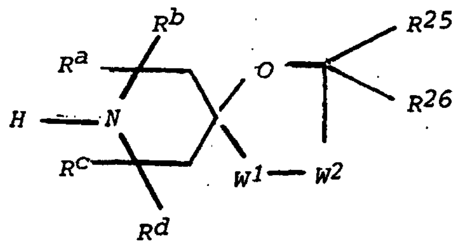

(XIII)

dans laquelle $W^1$ et $W^2$ sont différents et représentent — C(O) — ou — NH —, $R^{25}$ et $R^{26}$, identiques ou différents, représentent un atome d'hydrogène, un radical alkyle, linéaire ou ramifié, ayant un nombre d'atomes de carbone allant de 1 à 18, un radical phényle, un radical naphtyle, un radical phénylalkyle, $R^{25}$ et $R^{26}$ peuvent également former ensemble et avec l'atome de carbone qui les porte un cycloalkyle ayant un nombre d'atomes de carbone allant de 5 à 12 ou un radical 2,2,6,6-tétraméthylpipéridinyle ;

- les polyamines secondaires représentées par la formule générale (XIV) :

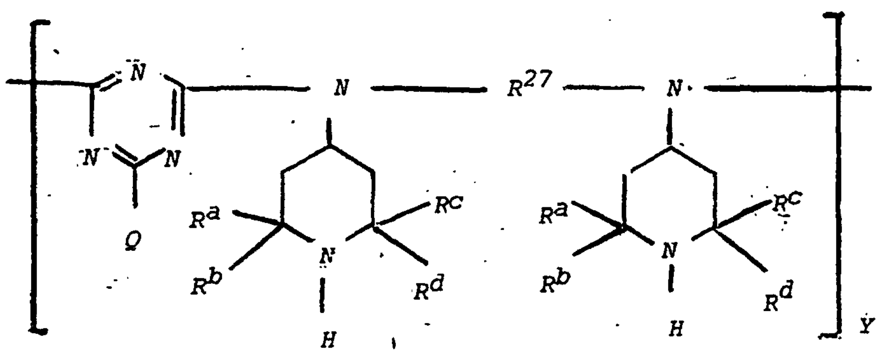

(XIV)

dans laquelle y = 1 à 20, $R^{27}$ représente un radical alkylène ayant un nombre d'atomes de carbone allant de 2 à 12 qui peut être interrompu par un — O— ou — $NR^{28}$ —, $R^{28}$ désignant un atome d'hydrogène, un radical alkyle ayant un nombre d'atomes de carbone allant de 1 à 12, un radical cycloalkyle, Q représente un radical — $OR^{29}$, — $NHR^{30}$, — $NR^{30}R^{31}$ ou $R^{29}$ représente un radical alkyle, linéaire ou ramifié ayant un nombre d'atomes de carbone allant de 1 à 12, un radical alcoxyalkyle en $C_3$ — $C_{12}$, un radical cyclohexyle, un radical benzyle, un radical phényle, un radical tolyle, un reste 2,2,6,6-tétramethylpipéridinyle, $R^{30}$ et $R^{31}$ ont la même signification que $R^{29}$, $R^{30}$ et $R^{31}$ peuvent également former ensemble et avec l'atome d'azote qui les porte un radical hétérocyclique à 5,6 ou 7 chaînons pouvant contenir en outre un oxygène ;

- la polyamine secondaire représentée par la formule (XV) :

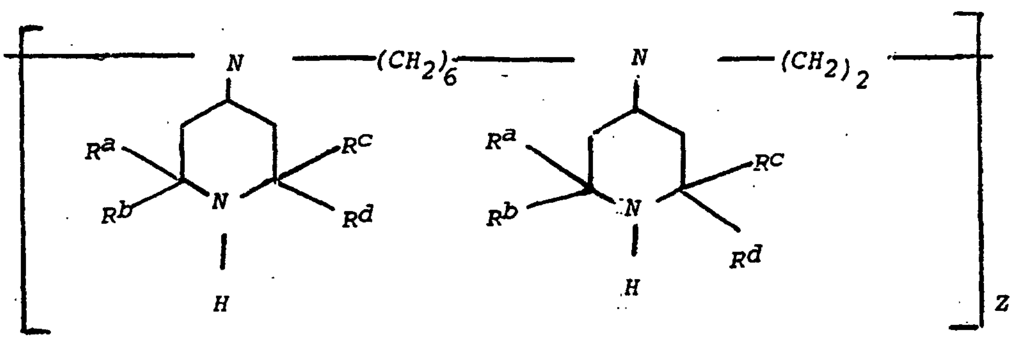

(XV)

dans laquelle z = 1 à 200.

La présente invention s'applique tout particulièrement aux amines secondaires ci-après :

- la 2,2,6,6-tétraméthylpipéridine (formule (I) dans laquelle X = $-CH_2-$ et $R^a = R^b = R^c = R^d = -CH_3$),
- la 4-hydroxy-2,2,6,6-tétraméthylpipéridine (formule (I) dans laquelle X = > CHZ avec Z = OH et $R^a = R^b = R^c = R^d = -CH_3$),
- la 4-oxo-2,2,6,6-tétraméthylpipéridine (formule (I) dans laquelle X = -C(O)- et $R^a = R^b = R^c = R^d = -CH_3$),
- le sébaçate de bis(2,2,6,6-tétraméthylpipéridine) (formule- (II) dans laquelle Y = $- OC(O)\ (CH_2)_8\ C(O)O$) et $R^a=R^b=R^c=R^d= -CH_3$),
- la 2,2,3,4,5,5-hexaméthylpyrrolidine (formule (III) dans laquelle $R^4 = R^5 = —CH_3$, et $R^a = R^b = R^c = R^d = -CH_3$),
- la 2,2,4,5,5-pentamethyl-3-pyrrolidinylcarboxamide (formule (III) dans laquelle $R^4 = -CH_3$, $R^5 = —C(O)NH_2$ et $R^a=R^b=R^c=R^d= -CH_3$),
- la 2,2,4,5,5-pentamethyl-3-pyrrolinylcarboxamide (formule- (IV) dans laquelle $R^6 = —CH_3$, $R^7 = —C(O)NH_2$ et $R^a=R^b=R^c=R^d= -CH_3$),
- les aminophosphates de formule (XII) dans laquelle $R^{20}$=—H, $R^{21}=(CH_3)_3C-$, $C_6H_{11}—$, $(CH_3)_2CH—$, $R^{22} = (CH_3)_3C—$, $C_6H_{11}$, $R^{23}=R^{24}=C_2H_5O-$, isopropoxy. Tout particulièrement, on utilisera le 2,2-diméthyl-1-(1,1-diméthylamino)propyl phosphonate de diéthyle; ($R^{20}$ = H, $R^{21} = R^{22} = (CH_3)_3C—$, $R^{23}=R^{24} = C_2H_5O-$) ;
- l'hexaméthyl-2,2,7,7,9,9-oxa-1-diaza-4,8-oxo-3-spiro[4.5]décane (formule XIII) dans laquelle $R^{25} = R^{26} = —CH_3$, W1 =—NH— et W2 =—C(O)—) ;
- la tétraméthyl —2,2,4,4-diaza-3,20-oxa-7oxo-21-dispiro[5.1.11.2]-heneicosane commercialisé par la société Hoechst sous la désignation HOSTAVIN® N-20 (formule (XIII) dans laquelle $R^{25}$ et $R^{26}$ forment un cycle —$(CH_2)_{11}$—, W1=—C(O)—, W2=—NH— et $R^a=R^b=R^c=R^d= -CH_3$) ;
- la polyamine de formule (XIV) dans laquelle $R^{27}$=—$(CH_2)_6$—, Q=NH-t$C_8H_{17}$ et $R^a=R^b=R^c=R^d= -CH_3$), commercialisée par la société CIBA-GEIGY sous la désignation CHIMASSORB® 944.

Les exemples qui suivent illustrent l'invention.
Les amines secondaires utilisées sont :

- la 2,2,6,6-tétraméthylpipéridine,
- le sébacate de bis(2,2,6,6-tétraméthylpipéridine),
- le 2,2-diméthyl-1-(1,1-diméthyléthylamino)propyl phosphonate de diéthyle obtenu selon une méthode décrite dans la demande internationale WO 96/24620,
- le CHIMASSORB® 944 de masse moléculaire en poids égale à environ 3000.

Les nitroxydes obtenus sont identifiés par chromatographie en phase vapeur (CPV), par leurs points de fusion et par spectrométrie de masse (SM). Les spectres de masse magnétique ont été réalisés sur un spectromètre de masse AUTOSPEC® de MICROMASS équipé avec une source d'ionisation à pression atmosphérique (API). Les spectres de masse ont été obtenus par accumulation de x spectres (x doit correspondre au temps d'injection boucle de 20 µl directement dans la source API) .

**EXEMPLE 1**

**Préparation du (2,2,6,6-tétraméthylpipéridine)-N-oxyde (TEMPO) :**

Dans un ballon de 100 ml muni de 2 ampoules de coulée, d'un réfrigérant, d'une sonde pH métrique et d'une agitation, on prépare sous agitation une solution organique constituée par 5 g de 2,2,6,6-tétraméthylpipéridine (soit 0,0354 mole) dissout dans 20 ml de dichlorométhane. On ajoute ensuite à cette solution 20 ml d'eau de manière à avoir un système biphasique. Ensuite, on introduit (sous agitation) lentement et simultanément 10,8 g d'une solution d'acide peracétique à 40 % en poids dans l'acide acétique et une solution aqueuse de $K_2CO_3$ à 35 % en poids. La quantité molaire d'acide péracétique introduite est de 0,0568 mole, ce qui correspond à un rapport molaire acide peracétique/amine de 1,6.
La quantité de solution aqueuse de $K_2CO_3$ est ajustée de telle sorte qu'au cours de l'addition, le pH de la phase aqueuse du milieu biphasique soit maintenu entre 7,2 et 7,5.
20 minutes après l'addition, on constate par chromatographie en phase vapeur (CPV) la disparition totale de l'amine et la formation du TEMPO.

La réaction est arrêtée et on ajoute de la solution de $K_2CO_3$ de manière à obtenir un pH égal à 9 puis on extrait le TEMPO, de couleur rouge, au moyen de $CH_2Cl_2$.

Par évaporation du solvant, on obtient 4,9 g de TEMPO présentant un point de fusion égal à 36°C. La pureté du TEMPO est vérifiée par CPV par rapport à un échantillon de produit pur (pureté supérieure à 99 %) commercialisé par la Société ALDRICH.

Spectre de masse (m/e) : 157 (M+1).

Le rendement en TEMPO par rapport à l'amine mise en oeuvre est de 88 %.

**EXEMPLE 2**

**Préparation du sébaçate de bis-(2,2,6,6-tétraméthyl pipéridine-N-oxyde) :**

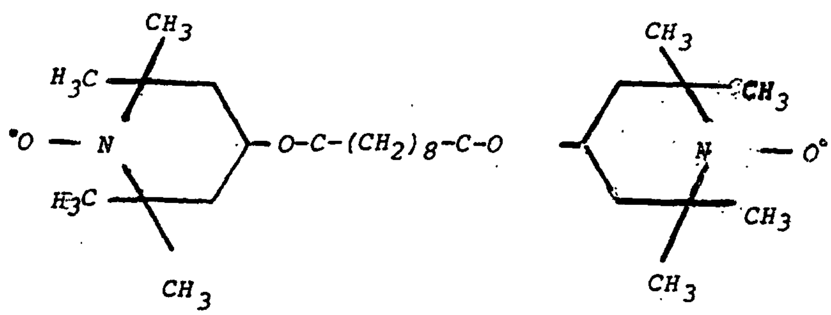

Dans un montage similaire à celui décrit dans l'exemple 1 (volume du réacteur = 500 ml), on prépare sous agitation une solution organique consitutée par 48 g de sébaçate de bis-(2,2,6,6-tétraméthylpipéridine), soit 0,10 mole, dans 200 ml de $CH_2Cl_2$. On ajoute ensuite à cette solution 50 ml d'eau.

On maintient le mélange biphasique ainsi obtenu sous vive agitation.

On introduit ensuite, sous agitation, lentement et simultanément 71,2 ml d'une solution d'acide peracétique à 32 % en poids dans l'acide acétique et une solution aqueuse de $K_2CO_3$ à 35 % en poids de manière à maintenir le pH de la phase aqueuse du milieu biphasique entre 7,2 et 7,5.

Au bout d'une heure, on arrête la réaction, en élevant le pH à 8,2.

On laisse décanter et on récupère le nitroxyde par extraction au $CH_2Cl_2$.

Par évaporation du $CH_2Cl_2$, on obtient 49,6 g d'un solide rouge présentant un point de fusion de 99,8°C. Pour ce composé Rozantsev, E.G. et al. (Izv. Akad. Nauk ; S.S.S.R. Ser Khim. 572, 1965) donnent un point de fusion de 101°C et la fiche de sécurité du produit commercialisé par la Société CIBA-GEIGY sous la désignation CXA 5415 donne un intervalle de fusion de 95°C - 102°C.

Spectre de masse (m/e) : 511 (M +1); 528 (N + 18)

Le rendement en nitroxyde par rapport à la diamine mise en oeuvre est de 97,3 %.

**EXEMPLE 3**

**Obtention du N-tertiobutyl-1-diéthylphosphono-2,2-diméthylpropylnitro**xyde (ci-après nitroxyde β-phosphoré) par oxydation du 2,2-diméthyl-1-(1,1-diméthyléthylamino)propyl phosphonate de diéthyle (ci-après aminophosphonate) :

On dilue 5 g d'aminophosphonate (0,017 mole) dans 100 ml d'acétate d'éthyle dans un réacteur muni de deux ampoules de coulée d'une agitation mécanique et d'une sonde pHmétrique. On ajoute 60 ml d'eau et on maintient le mélange sous une vive agitation. Ensuite, on introduit, sous agitation, lentement et simultanément 13,77 g d'acide perpropionique à 20 % en poids (0,0307 mole) dans le propionate d'éthyle et une solution aqueuse de $K_2CO_3$ à 8,5 % en poids (13 g de $K_2CO_3$ dans 140 ml d'eau) de manière à maintenir le pH de la phase aqueuse du milieu biphasique entre 5 et 7. On observe un dégagement gazeux ($CO_2$). La solution jaunit progressivement et on contrôle régulièrement le taux d'oxygène actif dans chaque phase. Au bout de 16 heures, la phase organique est orange et le dégagement gazeux a cessé totalement. On récupère par décantation la phase organique dont on évapore le solvant. Le nitroxyde brut se présente sous la forme d'une huile orange foncé (4,6 g) dont la pureté est égale à 62,5 %. Le rendement en nitroxyde β-phosphoré est de 57,5 %.

Spectre de masse (m/e) : 295 (M + 1)

**EXEMPLE 4**

**Oxydation du CHIMASSORB® 944 selon la présente invention :**

Dans l'appareillage décrit dans l'exemple 2 on solubilise 11,1 g de CHIMASSORB® 944 dans 111 g de $CH_2Cl_2$.
On ajoute ensuite à la solution obtenue 50 ml d'eau et on maintient le milieu biphasique sous agitation.
On ajoute, à température ambiante et sous agitation simultanément et en 20 minutes, 23,4 g d'acide peracétique (en solution à 40 % en poids dans l'acide acétique) et 57,6 g de solution aqueuse de $K_2CO_3$ à 35 % en poids.
Cette addition se fait de telle sorte que le pH de la phase aqueuse soit maintenu entre 7 et 7,5.
La réaction est exothermique malgré un dégagement de $CO_2$.
En fin d'addition, le milieu, devenu rouge-orange, est maintenu 2 heures sous agitation.
Ensuite, on arrête l'agitation et on laisse, décanter 15 heures.
La phase organique est récupérée et on évapore le $CH_2Cl_2$ sous pression réduite. On obtient 10,7 g d'un solide rouge paramagnétique. Rendement : 91 %.

## Revendications

1. Procédé de préparation de radicaux N-oxyde d'amines secondaires (radicaux nitroxydes) à partir des amines secondaires correspondantes, **caractérisé en ce que** l'on effectue les étapes suivantes :

   a/ on solubilise l'amine secondaire avec un solvant organique, non miscible à l'eau, puis on ajoute de l'eau,
   b/ on ajoute ensuite simultanément et sous, forte agitation au milieu biphasique ainsi obtenu, une quantité de peracide aliphatique selon un rapport molaire peracide / amine secondaire allant de 1,5 à 2,5 et une quantité suffisante d'une solution aqueuse basique pour obtenir un pH de la phase aqueuse du milieu biphasique allant de 4 à 12, à une température comprise entre -10°C et +40°C, jusqu'à conversion complète de l'amine secondaire, puis
   c/ on récupère la phase organique par simple décantation, et on isole le nitroxyde par évaporation du solvant organique sous pression réduite.

2. Procédé selon la revendication 1, **caractérisé en ce que** le rapport molaire peracide / amine secondaire va de 1,5 à 2.

3. Procédé selon la revendication 1 ou 2, **caractérisé en ce que** le pH de la phase aqueuse du milieu biphasique va de 5 à 9.

4. Procédé selon l'une quelconque des revendications 1 à 3, **caractérisé en ce que** la température est comprise entre -5°C et + 30°C,

5. Procédé selon l'une quelconque des revendications 1 à 4, **caractérisé en ce que** le solvant organique est choisi parmi les hydrocarbures aliphatiques ou cycloaliphatiques, les hydrocarbures aromatiques, les hydrocarbures chlorés, les esters d'acides aliphatiques.

6. Procédé selon la revendication 5, **caractérisé en ce que** les hydrocarbures aliphatiques sont le pentane, l'hexane ou l'heptane, les hydrocarbures chlorés sont le chlorure de méthylène ou le 1,2-dichloroéthane, les esters d'acides aliphatiques sont l'acétate d'éthyle ou le propionate d'éthyle.

7. Procédé selon la revendication 1, **caractérisé en ce que** le peracide aliphatique est l'acide peracétique, l'acide perpropionique ou l'acide perbutanoïque.

8. Procédé selon la revendication 1, **caractérisé en ce que** la solution aqueuse basique est une solution aqueuse d'un carbonate ou d'un hydrogénocarbonate d'un métal alcalin ou d'un métal alcalino-terreux.

9. Procédé selon la revendication 8, **caractérisé en ce que** le carbonate ou l'hydrogénocarbonate d'un métal alcalin est $NaHCO_3$, $KHCO_3$,$K_2CO_3$ ou $Na_2CO_3$.

**10.** Procédé selon l'une quelconque des revendications 1 à 9 dans lequel l'amine secondaire est choisie parmi les composés représentés par les formules ci-après dans lesquelles $R^a$, $R^b$, $R^c$ et $R^d$, identiques ou différents, représentent un radical alkyle, linéaire ou ramifié, ayant un nombre d'atomes de carbone allant de 1 à 10, ou $R^a$ et $R^b$ forment avec l'atome de carbone qui les porte, ou $R^c$ et $R^d$ forment avec l'atome de carbone qui les porte un cycloalcyle ayant un nombre d'atomes de carbone allant de 3 à 10, un reste spiranique, cholestanique ou androstanique :

- les composés de formule (I) :

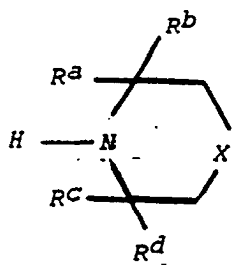

(I)

dans laquelle X représente un radical divalent choisi parmi les radicaux suivants: $>CH_2$ ; $>C=O$ ; —O— ;

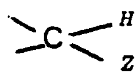

dans lequel Z représente un reste monovalent choisi parmi —CN, —NHR, —OR, —N =C(R)$_2$ dans lesquels R représente un atome d'hydrogène, un radical alkyle, linéaire ou ramifié ayant un nombre d'atomes de carbone allant de 1 à 10, un radical benzyle, un radical phényle, —CN, —C(O)$R^1$ ; $>C=N-R^1$, $>C(OR^1)_2$ ou $R^1$ a la même signification que R;

- les composés de formule (II) :

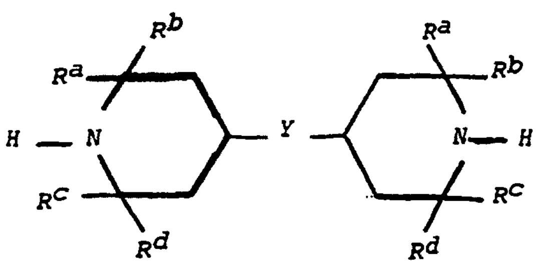

(II)

dans laquelle Y représente un reste divalent choisi parmi :

—OC(O)—$(CR^2R^3)_n$—C(O)O—,

—NH—$(CR^2R^3)_n$NH—,

—NHC(O)—$(CR^2R^3)_n$—C(O)NH,

—S—, —O—, $R^2$ et $R^3$, identiques ou différents, représentent un atome d'hydrogène, un radical alkyle, linéaire ou ramifié, ayant un nombre d'atomes de carbone allant de 1 à 10, n représentent un nombre entier allant de 0 à 20 ;

- les composés de formule (III) :

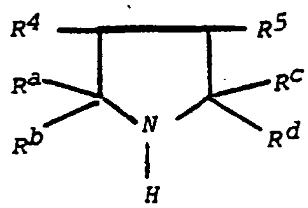

(III)

dans laquelle $R^4$ représente un atome d'hydrogène un radical alkyle, linéaire ou ramifié, ayant un nombre d'atomes de carbone allant de 1 à 10, $R^5$ représente un atome d'hydrogène $-CH_3$, $-C_2H_5$, $-CH_2OH$, $-C(O)NH_2$, $-OH$, $-CHO$ ;

- les composés de formule (IV) :

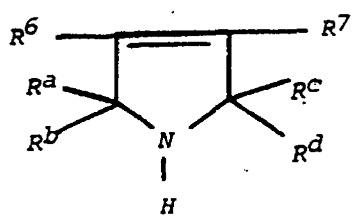

(IV)

dans laquelle $R^6$ a la même signification que $R^4$ et $R^7$ a la même signification que $R^5$,

- les 1,1,3,3-tétraméthyl-pyrrolopyridines de formule (V) :

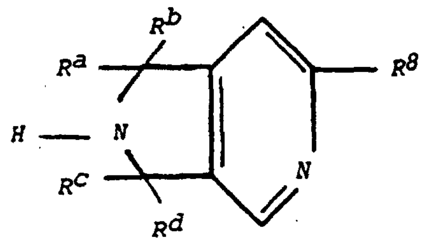

(V)

et les 1,1,3,3-tétraméthyl-2,3-dihydroisoindoles de formule (VI) :

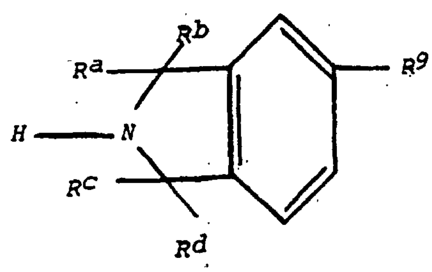

(VI)

dans lesquelles $R^8$ et $R^9$ représentent un atome d'hydrogène, un radical alkyle, linéaire ou ramifié, ayant un nombre d'atomes de carbone allant de 1 à 10 ;

- les composés de formule (VII) :

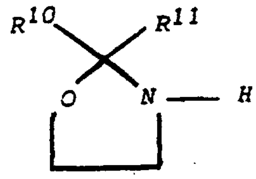

(VII)

dans laquelle $R^{10}$ et $R^{11}$, identiques ou différents, représentent un radical alkyle, linéaire ou ramifié, ayant un nombre d'atomes de carbone allant de 1 à 20, un radical carboxyalkyle $-(CH_2)mCO_2H$ dans lequel : m =1 à 20 ;

- les composés de formule (VIII) :

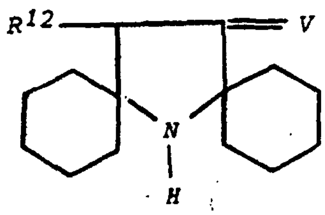

(VIII)

dans laquelle $R^{12}$ représente un atome d'hydrogène, un reste alkyle, linéaire ou ramifié ayant un nombre d'atomes de carbone allant de 1 à 20, un radical—C(O)$NH_2$, V = 0 ; S, NH ;

- les composés de formule (IX) :

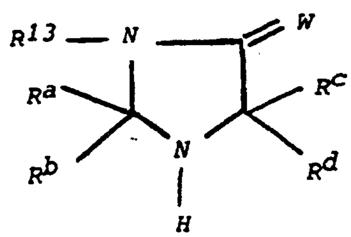

(IX)

dans laquelle $R^{13}$ a la même signification que $R^{12}$ et W a la même signification que V de la formule (VIII) ;

- les composés de formule (X) :

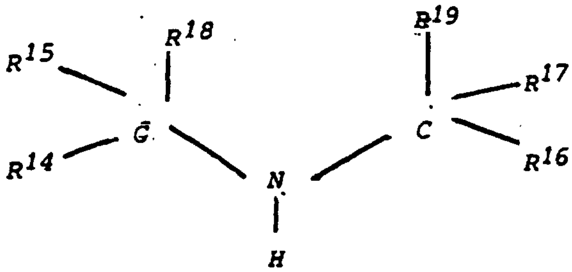

(X)

dans lesquels $R^{14}$, $R^{15}$, $R^{16}$, $R^{17}$, $R^{18}$, et $R^{19}$, identiques. ou différents représentent un radical alkyle, linéaire ou ramifié, ayant un nombre d'atomes de carbone allant de 1 à 20, un radical cycloalkyle ayant au moins 3 atomes de carbone, un radical phényle, un radical benzyle ; ou bien $R^{14}$ et $R^{15}$ forment avec l'atome de carbone qui les porte, ou bien $R^{16}$ et $R^{17}$ forment avec l'atome de carbone qui les porte, un cycloalkyle ayant un nombre d'atomes de carbone allant de 3 à 10 ;

- la 1,2,3,6-tétrahydro-2,2,6,6-tétraméthylpyridine :
- la 3,3-diméthyl-1-oxa-4-azaspiro[4.5]décane :
- la 2,3,3,5,5-pentaméthylmorpholine :

- la 3,3,5,5-tétraméthyl-2-méthylènemorpholine,
- le N-(2,2,6,6-tétraméthylpipéridyl-4)-ε-caprolactame :
- le 4,4'diméthylspiro(5α-cholestane-3,2'oxazolidine):
- les composés de formule (XI) :

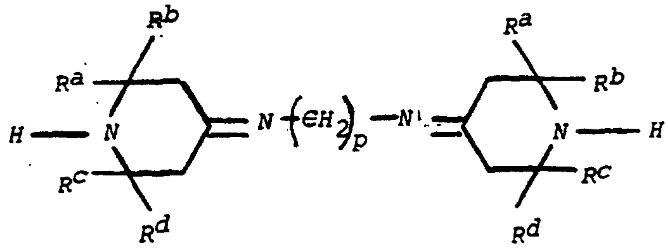

(XI)

avec p =0 à 20.

- les composés de formule (XII) :

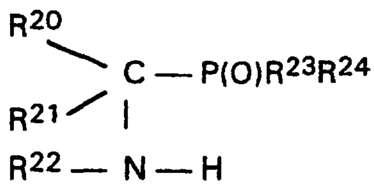

(XII)

dans laquelle dans laquelle $R^{20}$ et $R^{21}$, identiques ou différents, représentent un atome d'hydrogène, un radical alkyle linéaire, ramifié ou cyclique ayant un nombre d'atomes de carbone allant de 1 à 10, un radical aryle, un radical aralkyle, ou bien $R^{20}$ et $R^{21}$ sont reliés entre eux de façon à former un cycle incluant l'atome de carbone portant lesdits radicaux $R^{20}$ et $R^{21}$, ledit cycle ayant un nombre d'atomes de carbone, incluant le carbone porteur des radicaux $R^{20}$ et $R^{21}$, allant de 3 à 8 ; $R^{22}$ représente un radical hydrocarboné, linéaire ou ramifié, saturé ou insaturé, pouvant comprendre au moins un cycle, ledit radical ayant un nombre d'atomes de carbone allant de 1 à 30 ; $R^{23}$ et $R^{24}$, identiques ou différents, représentent un radical alkyle, linéaire ou ramifié, ayant un nombre d'atomes de carbone allant de 1 à 20, un radical cycloalkyle, aryle, alkoxyle, aryloxyle, aralkyloxyle, perfluoroalkyle, aralkyle, dialkyle. ou diarylamino, alkylarylamino, thioalkyle, ou bien $R^{23}$ et $R^{24}$ sont reliés entre eux de façon à former un cycle incluant l'atome de phosphore, ledit hétérocycle ayant un nombre d'atomes de carbone allant de 2 à 4 et peut contenir en outre 1 ou plusieurs atomes d'oxygène, de soufre ou d'azote ;

- les oxa-1-diaza-oxo-spirodécanes de formule (XIII) :

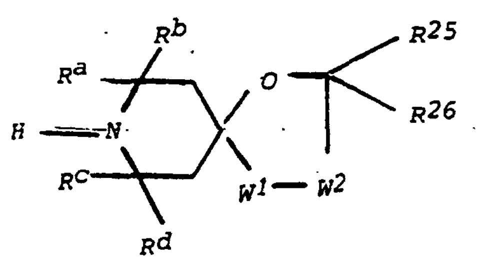

(XIII)

dans laquelle $W^1$ et $W^2$ sont différents et représentent — C(O) — ou — NH —, $R^{25}$ et $R^{26}$, identiques ou différents, représentent un atome d'hydrogène, un radical alkyle, linéaire ou ramifié, ayant un nombre d'atomes de carbone allant de 1 à 18, un radical phényle, un radical naphtyle, un radical phénylalkyle, ou bien $R^{25}$ et $R^{26}$ forment avec l'atome de carbone qui les porte un cycloalkyle ayant un nombre d'atomes de carbone allant de 5 à 12 ou un radical 2,2,6,6-tétraméthylpipéridinyle ;

- les polyamines secondaires représentées par la formule générale (XIV) :

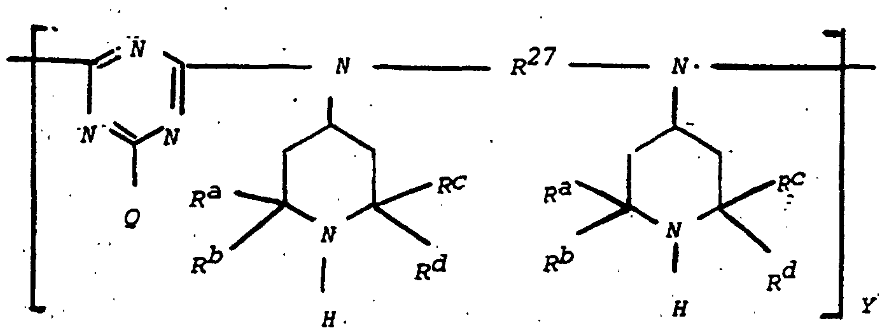

(XIV)

dans laquelle y = 1 à 20, $R^{27}$ représente un radical alkylène ayant un nombre d'atomes de carbone allant de 2 à 12 qui peut être interrompu par un — O— ou — $NR^{28}$—, $R^{28}$ désignant un atome d'hydrogène, un radical alkyle ayant un nombre d'atomes de carbone allant de 1 à 12, un radical cycloalkyle, Q représente un radical — $OR^{29}$, — $NHR^{30}$, —$NR^{30}R^{31}$ ou $R^{29}$ représente un radical alkyle, linéaire ou ramifié ayant un nombre d'atomes de carbone allant de 1 à 12, un radical alcoxyalkyle en $C_3$ — $C_{12}$, un radical cyclohexyle, un radical benzyle, un radical phényle, un radical tolyle, un reste 2,2,6,6-tétraméthylpipéridinyle, $R^{30}$ et $R^{31}$ ont la même signification que $R^{29}$, ou bien $R^{30}$ et $R^{31}$ forment avec l'atome d'azote qui les porte un radical hétérocyclique à 5,6 ou 7 chaînons pouvant contenir en outre un oxygène ;
- la polyamine secondaire représentée par la formule (XV) :

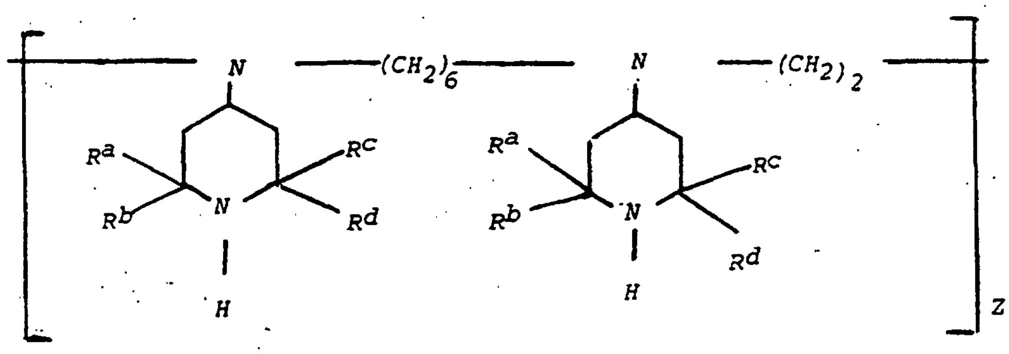

(XV)

dans laquelle z = 1 à 200.

**11.** Procédé selon la revendication 10, **caractérisé en ce que** l'amine secondaire est :

- la 2,2,6,6-tétraméthylpipéridine (formule (I) dans laquelle X = $-CH_2-$ et $R^a = R^b = R^c = R^d = -CH_3$),
- la 4-hydroxy-2,2,6,6-tétraméthylpipéridine (formulé (I) dans laquelle X = > CHZ avec Z = OH et $R^a = R^b = R^c = R^d = -CH_3$),
- la 4-oxo-2,2,6,6-tétraméthylpipéridine (formule (I) dans laquelle X = -C(O)- et $R^a = R^b = R^c = R^d = -CH_3$),
- le sébaçate de bis(2,2,6,6-tétraméthylpipéridine) (formule (II) dans laquelle Y = —OC(O) $(CH_2)_8$ C(O)O) et $R^a=R^b=R^c=R^d= -CH_3$),
- la 2,2,3,4,5,5-hexaméthylpyrrolidine (formule (III) dans laquelle $R^4 = R^5 = —CH_3$) et $R^a = R^b = R^c = R^d = -CH_3$),
- la 2,2,4,5,5-pentamethyl-3-pyrrolidinylcarboxamide (formule (III) dans laquelle $R^4 = CH_3$, $R^5$ = — $C(O)NH_2$ et $R^a=R^b=R^c=R^d= -CH_3$),
- la 2,2,4,5,5-pentamethyl-3-pyrrolinylcarboxamide (formule (IV) dans laquelle $R^6 = —CH_3$, $R^7 = —C(O)NH_2$ et $R^a=R^b=R^c=R^d= -CH_3$),
- le 2,2-diméthyl-1-(1,1-diméthylamino)propyl phosphonate de diéthyle, ($R^{20}$ = -H, $R^{21} = R^{22} = (CH_3)_3C—$,

$R^{23}=R^{24}=C_2H_5O$-) ;

- l'hexaméthyl-2,2,7,7,9,9-oxa-1-diaza-4,8-oxo-3-spiro[4.5]décane (formule XIII) dans laquelle $R^{25} = R^{26}$ =—$CH_3$, W1 =—NH— et W2 =—C(O)—),
- la tétraméthyl —2,2,4,4-diaza-3,20-oxa-7oxo-21-dispiro[5.1.11.2]heneicosane commercialisé par la société Hoechst sous la désignation HOSTAVIN® N-20 (formule (XIII) dans laquelle $R^{25}$ et $R^{26}$ forment un cycle —$(CH_2)_{11}$—, W1 =—C(O)—, W2 =—NH— et $R^a=R^b=R^c=R^d= -CH_3$),
- la polyamine de formule (XIV) dans laquelle $R^{27}$ =—$(CH_2)_6$—, Q=NH-t$C_8H_{17}$ et $R^a=R^b=R^c=R^d= -CH_3$), commercialisée par la société CIBA-GEIGY sous la désignation CHIMASSORB® 944.

**Patentansprüche**

1. Verfahren zur Herstellung von sek.-Amin-N-oxid-Radikalen (Nitroxidradikalen) aus den entsprechenden sekundären Aminen, **dadurch gekennzeichnet, daß** man:

   a) das sekundäre Amin in einem nicht wassermischbaren organischen Lösungsmittel löst und dann Wasser zugibt,

   b) das so erhaltene zweiphasige Medium unter kräftigem Rühren bei einer Temperatur zwischen - 10°C und +40°C gleichzeitig mit einer Menge an aliphatischer Persäure in einem Molverhältnis von Persäure zu sekundärem Amin von 1,5 bis 2,5 und einer so großen Menge einer basischen wäßrigen Lösung, daß man einen pH-Wert der wäßrigen Phase des zweiphasigen Mediums von 4 bis 12 erhält, versetzt, bis das sekundäre Amin vollständig umgewandelt ist;

   c) die organische Phase durch einfaches Absetzenlassen zurückgewinnt und das Nitroxid durch Abziehen des organischen Lösungsmittels unter vermindertem Druck isoliert.

2. Verfahren nach Anspruch 1, **dadurch gekennzeichnet, daß** das Molverhältnis von Persäure zu sekundärem Amin 1,5 bis 2 beträgt.

3. Verfahren nach Anspruch 1 oder 2, **dadurch gekennzeichnet, daß** der pH-Wert der wäßrigen Phase des zweiphasigen Mediums 5 bis 9 beträgt.

4. Verfahren nach einem der Ansprüche 1 bis 3, **dadurch gekennzeichnet, daß** die Temperatur zwischen -5°C und +30°C liegt.

5. Verfahren nach einem der Ansprüche 1 bis 4, **dadurch gekennzeichnet, daß** man das organische Lösungsmittel unter aliphatischen und cycloaliphatischen Kohlenwasserstoffen, aromatischen Kohlenwasserstoffen, chlorierten Kohlenwasserstoffen und Estern aliphatischer Säuren auswählt.

6. verfahren nach Anspruch 5, **dadurch gekennzeichnet, daß** es sich bei den aliphatischen Kohlenwasserstoffen um Pentan, Hexan oder Heptan handelt, bei den chlorierten Kohlenwasserstoffen um Methylenchlorid oder 1,2-Dichlorethan handelt und bei den Estern aliphatischer Säuren um Essigsäureethylester oder Propiosäureethylester handelt.

7. Verfahren nach Anspruch 1, **dadurch gekennzeichnet, daß** es sich bei der Persäure um Peressigsäure, Perpropionsäure oder Perbutansäure handelt.

8. Verfahren nach Anspruch 1, **dadurch gekennzeichnet, daß** es sich bei der basischen wäßrigen Lösung um eine wäßrige Lösung eines Alkalimetall- oder Erdalkalimetallcarbonats oder -hydrogencarbonats handelt.

9. Verfahren nach Anspruch 8, **dadurch gekennzeichnet, daß** es sich bei dem Alkalimetallhydrogencarbonat oder -carbonat um $NaHCO_3$, $KHCO_3$, $K_2CO_3$ oder $Na_2CO_3$ handelt.

10. Verfahren nach einem der Ansprüche 1 bis 9, bei dem man das sekundäre Amin unter den Verbindungen der nachstehenden Formeln auswählt, in denen $R^a$, $R^b$, $R^c$ und $R^d$ gleich oder verschieden sind und für einen linearen oder verzweigten Alkylrest mit 1 bis 10 Kohlenstoffatomen stehen oder $R^a$ und $R^b$ mit dem Kohlenstoffatom, an das sie gebunden sind, oder $R^c$ und $R^d$ mit dem Kohlenstoffatom, an das sie gebunden sind, einen Cycloalkylrest

mit 3 bis 10 Kohlenstoffatomen oder einen Spiran-, Cholestanoder Androstanrest bilden:

- Verbindungen der Formel (I):

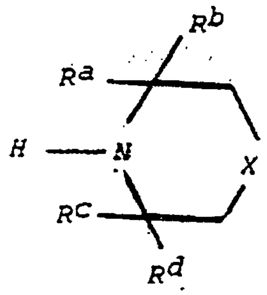

(I)

worin X für einen unter den folgenden Resten ausgewählten zweiwertigen Rest steht: $>CH_2$ ; $>C=O$ ; —O— ;

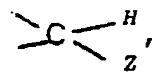

worin Z für einen unter -CN, -NHR, -OR und $N=C(R)_2$, worin R ein Wasserstoffatom, einen linearen oder verzweigten Alkylrest mit 1 bis 10 Kohlenstoffatomen, einen Benzylrest, einen Phenylrest, -CN oder $-C(O)R^1$ bedeutet, ausgewählten einwertigen Rest steht, —CN, $—C(O)R^1$ ; $>C=N—R^1$, $>C(OR^1)_2$, worin $R^1$ die gleiche Bedeutung wie R hat;

- Verbindungen der Formel (II):

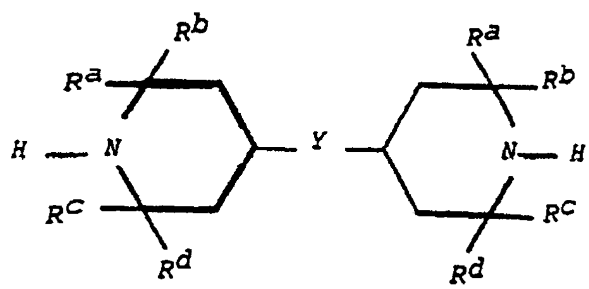

(II)

worin Y für einen unter:
- $OC(O)-(CR^2R^3)_n-C(O)O-$,
- $NH-(CR^2R^3)_n-NH-$,
$-NHC(O)-(CR^2R^3)_n-C(O)NH-$,
- S- und -O- ausgewählten zweiwertigen Rest steht, $R^2$ und $R^3$ gleich oder verschieden sind und für ein Waserstoffatom oder einen linearen oder verzweigten Alkylrest mit 1 bis 10 Kohlenstoffatomen stehen und n für eine ganze Zahl von 0 bis 20 steht;

- Verbindungen der Formel (III):

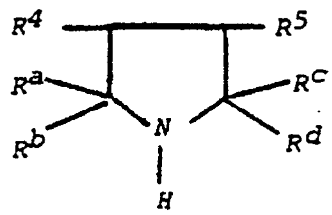

(III)

worin $R^4$ für ein Wasserstoffatom oder einen linearen oder verzweigten Alkylrest mit 1 bis 10 Kohlenstoffatomen steht und $R^5$ für ein Wasserstoffatom, $-CH_3$, $-C_2H_5$, $-CH_2OH$, $-C(O)NH_2$, $-OH$ oder $-CHO$ steht;

- Verbindungen der Formel (IV):

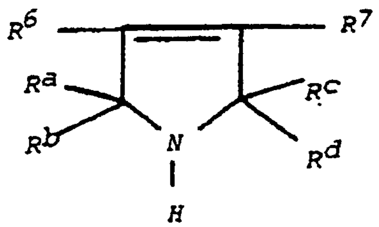

(IV)

worin $R^6$ die gleiche Bedeutung wie $R^4$ hat und $R^7$ die gleiche Bedeutung wie $R^5$ hat;

- 1,1,3,3-Tetramethylpyrrolopyridine der Formel (V)

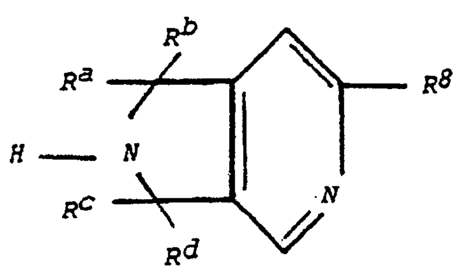

(V)

und 1,1,3,3-Tetramethyl-2,3-dihydroisoindole der Formel (VI):

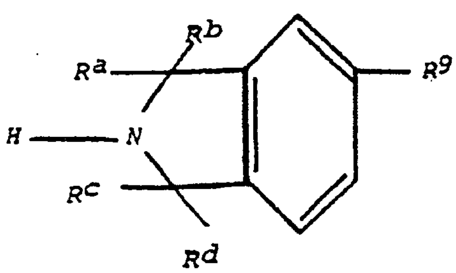

(VI)

worin $R^8$ und $R^9$ für ein Wasserstoffatom oder einen linearen oder verzweigten Alkylrest mit 1 bis 10 Kohlenstoffatomen stehen;

- Verbindungen der Formel (VII):

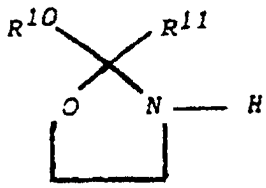

(VII)

worin $R^{10}$ und $R^{11}$ gleich oder verschieden sind und für einen linearen oder verzweigten Alkylrest mit 1 bis 20 Kohlenstoffatomen oder einen Carboxylalkylrest $-(CH_2)_mCO_2H$ mit m = 1 bis 20 stehen;

- Verbindungen der Formel (VIII):

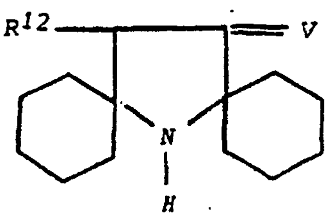

(VIII)

worin $R^{12}$ für ein Wasserstoffatom oder einen linearen oder verzweigten Alkylrest mit 1 bis 20 Kohlenstoffatomen oder einen $-C(O)NH_2$-Rest steht und V für O, S oder NH steht;

- Verbindungen der Formel (IX):

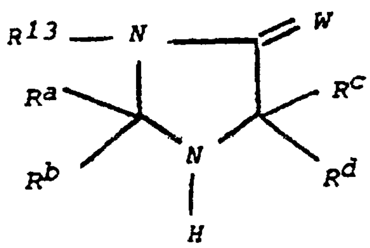

(IX)

worin $R^{13}$ die gleiche Bedeutung wie $R^{12}$ hat und W die gleiche Bedeutung wie V in Formel (VIII) hat;

- Verbindungen der Formel (X):

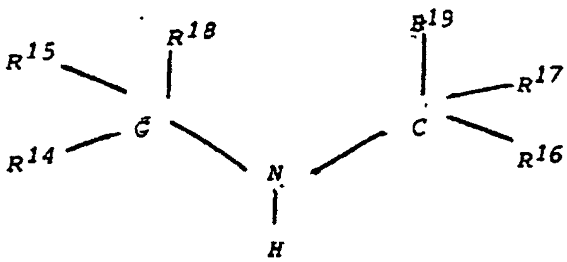

(X)

worin $R^{14}$, $R^{15}$, $R^{16}$, $R^{17}$, $R^{18}$ und $R^{19}$ gleich oder verschieden sind und für einen linearen oder verzweigten Alkylrest mit 1 bis 20 Kohlenstoffatomen, einen Cycloalkylrest mit mindestens 3 Kohlenstoffatomen, einen Phenylrest, oder einen Benzylrest stehen oder $R^{14}$ und $R^{15}$ mit dem Kohlenstoffatom, an das sie gebunden

sind, oder $R^{16}$ und $R^{17}$ mit dem Kohlenstoffatom, an das sie gebunden sind, einen Cycloalkylrest mit 3 bis 10 Kohlenstoffatomen bilden;

- 1,2,3,6-Tetrahydro-2,2,6,6-tetramethylpyridin;
- 3,3-Dimethyl-1-oxa-4-azaspiro[4.5]decan;
- 2,3,3,5,5-Pentamethylmorpholin;
- 3,3,5,5-Tetramethyl-2-methylenmorpholin;
- N-(2,2,6,6-Tetramethyl-4-piperidyl)-ε-caprolactam;
- 4,4'-Dimethylspiro(5α-cholestan-3,2'oxazolidin);
- Verbindungen der Formel (XI):

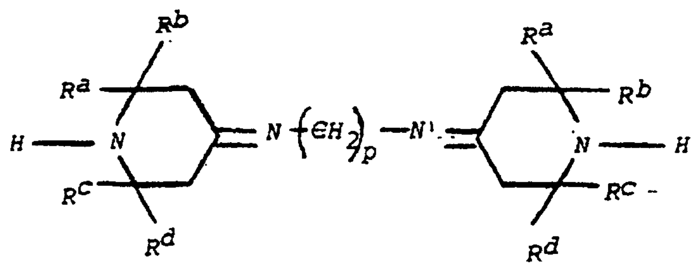

(XI)

mit p = 0 bis 20;

- Verbindungen der Formel (XII):

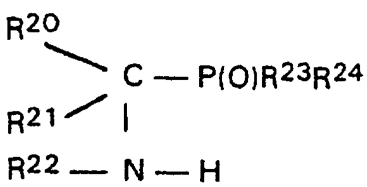

(XII)

worin $R^{20}$ und $R^{21}$ gleich oder verschieden sind und für ein Wasserstoffatom, einen linearen, verzweigten oder cyclischen Alkylrest mit 1 bis 10 Kohlenstoffatomen, einen Arylrest oder einen Aralkylrest mit 1 bis 10 Kohlenstoffatomen stehen oder auch so miteinander verbunden sind, daß sie mit dem die Reste $R^{20}$ und $R^{21}$ tragenden Kohlenstoffatom einen Ring bilden, der einschließlich des die Reste $R^{20}$ und $R^{21}$ tragenden Kohlenstoffatoms 3 bis 8 Kohlenstoffatome aufweist; $R^{22}$ für einen linearen oder verzweigten, gesättigten oder ungesättigten, gegebenenfalls mindestens einen Ring enthaltenden Kohlenwasserstoffrest mit 1 bis 30 Kohlenstoffatomen steht; $R^{23}$ und $R^{24}$ gleich oder verschieden sind und für einen linearen oder verzweigten Alkylrest mit 1 bis 20 Kohlenstoffatomen, einen Cycloalkyl-, Aryl-, Alkoxy-, Aryloxy-, Aralkoxy-, Perfluoralkyl-, Aralkyl-, Dialkyl- oder Diarylamino-, Alkylarylamino- oder Thioalkylrest stehen oder auch so miteinander verbunden sind, daß sie mit dem Phosphoratom einen Ring bilden, der 2 bis 4 Kohlenstoffatome aufweist und außerdem ein oder mehrere Sauerstoff-, Schwefel- oder Stickstoffatome enthalten kann;

- Oxa-1-diazaoxospirodecane der Formel (XIII):

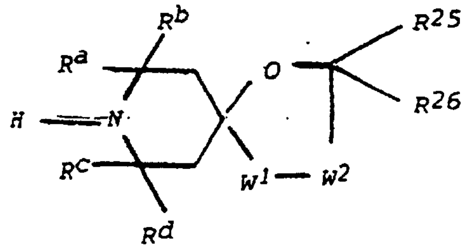

(XIII)

worin $W^1$ und $W^2$ gleich oder verschieden sind und für -C(O)- oder -NH- stehen, $R^{25}$ und $R^{26}$ gleich oder verschieden sind und für ein Wasserstoffatom, einen linearen oder verzweigten Alkylrest mit 1 bis 18 Kohlenstoffatomen, einen Phenylrest, einen Naphthylrest oder einen Phenylalkylrest stehen oder auch mit dem Kohlenstoffatom, an das sie gebunden sind, einen Cycloalkylring mit 5 bis 12 Kohlenstoffatomen oder einen 2,2,6,6-Tetramethylpiperidinrest bilden;

- sekundäre Polyamine der allgemeinen Formel (XIV):

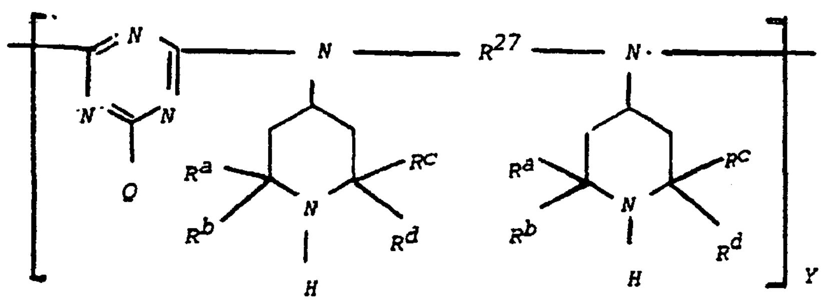

(XIV)

worin y für 1 bis 20 steht, $R^{27}$ für einen Alkylenrest mit 2 bis 12 Kohlenstoffatomen, die durch -O- oder -$NR^{28}$-, worin $R^{28}$ ein Wasserstoffatom, ein Alkylrest mit 1 bis 12 Kohlenstoffatomen oder ein Cycloalkylrest bedeutet, unterbrochen sein können, steht und Q für einen Rest -$OR^{29}$, -$NHR^{30}$, -$NR^{30}R^{31}$, worin $R^{29}$ ein linearer oder verzweigter Alkylrest mit 1 bis 12 Kohlenstoffatomen, ein $C_3$-$C_{12}$-Alkoxyalkylrest, ein Cyclohexylrest, ein Benzylrest, ein Phenylrest, ein Tolylrest oder ein 2,2,6,6-Tetramethylpiperidinylrest bedeutet und $R^{30}$ und $R^{31}$ die gleiche Bedeutung wie $R^{29}$ haben oder mit dem Stickstoffatom, an das sie gebunden sind, einen 5-, 6- oder 7-gliedrigen heterocyclischen Rest, der außerdem ein Sauerstoffatom enthalten kann, bilden, steht;

- das sekundäre Polyamin der Formel (XV):

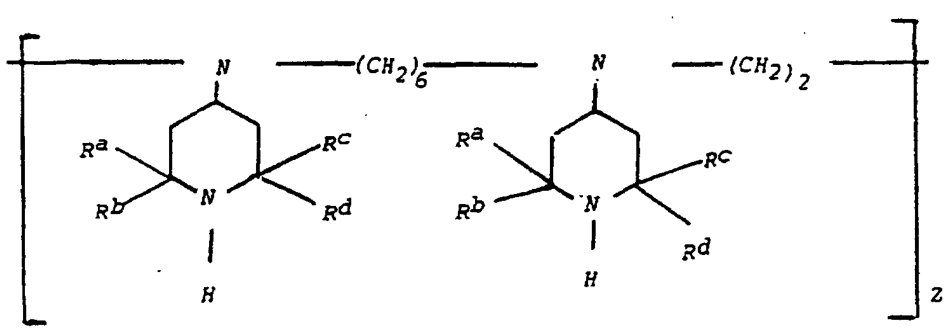

(XV)

worin z für 1 bis 200 steht.

**11.** Verfahren nach Anspruch 10, **dadurch gekennzeichnet, daß** es sich bei dem sekundären Amin um:

- 2,2,6,6-Tetramethylpiperidin (Formel (I), worin X = $-CH_2-$ und $R^a = R^b = R^c = R^d = -CH_3$),
- 4-Hydroxy-2,2,6,6-tetramethylpiperidin (Formel (I), worin X = >CHZ- mit Z = OH und $R^a = R^b = R^c = R^d = -CH_3$),
- 4-Oxo-2,2,6,6-tetramethylpiperidin (Formel (I), worin X = -C(O)- und $R^a = R^b = R^c = R^d = -CH_3$),
- Bis(2,2,6,6-tetramethylpiperidin)sebacat (Formel (II), worin Y = $-OC(O)(CH_2)_8C(O)O-$ und $R^a = R^b = R^c = R^d = -CH_3$),
- 2,2,3,4,5,5-Hexamethylpyrrolidin (Formel (III), worin $R^4 = R^5 = -CH_3$ und $R^a = R^b = R^c = R^d = -CH_3$),
- 2,2,4,5,5-Pentamethyl-3-pyrrolidinylcarbonsäureamid (Formel (III), worin $R^4 = CH_3$, $R^5 = -C(O)NH_2$ und $R^a = R^b = R^c = R^d = -CH_3$),
- 2,2,4,5,5-Pentamethyl-3-pyrrolinylcarbonsäureamid (Formel (IV), worin $R^6 = -CH_3$, $R^7 = -C(O)NH_2$ und $R^a = R^b = R^c = R^d = -CH_3$),
- 2,2-Dimethyl-1-(1,1-dimethylamino)propylphosphonsäurediethylester ($R^{20} = -H$, $R^{21} = R^{22} = (CH_3)_3C-$, $R^{23} = R^{24} = C_2H_5O-$);
- 2,2,7,7,9,9-Hexamethyl-1-oxa-4,8-diaza-3-oxospiro[4.5]decan (Formel (XIII), worin $R^{25} = R^{26}$ =
- $CH_3$, $W^1$ = -NH- und $W_2$ = -C(O)-),
- 2,2,4,4-Tetramethyl-3,20-diaza-7-oxa-21-oxodispiro[5.1.11.2]heneicosan, das von der Firma Hoechst unter der Bezeichnung HOSTAVIN® N-20 vertrieben wird (Formel (XIII), worin $R^{25}$ und $R^{26}$ einen $(-CH_2)_{11}$-Ring bilden, $W^1$ = -C(O)-, $W^2$ = -NH- und $R^a = R^b = R^c = R^d = -CH_3$),
- das Polyamin der Formel (XIV), worin $R^{27} = -(CH_2)_6-$, Q = $NH-tC_8H_{17}$ und $R^a = R^b = R^c = R^d = -CH_3$ und das von der Firma Ciba-Geigy unter der Bezeichnung CHIMASSORB® 944 vertrieben wird,

handelt.

## Claims

1. A process for preparing secondary amine N-oxide radicals (nitroxide radicals) from the corresponding secondary amines, **characterized in that** the following steps are carried out:

   a/ the secondary amine is dissolved with a water-immiscible organic solvent, and water is then added,
   b/ an amount of aliphatic peracid in a peracid/secondary amine molar ratio ranging from 1.5 to 2.5, and an amount of an aqueous basic solution sufficient to give the aqueous phase of the two-phase medium a pH ranging from 4 to 12, are then added simultaneously and with vigorous stirring to the two-phase medium thus obtained, at a temperature of between -10°C and +40°C, until conversion of the secondary amine is complete, and then
   c/ the organic phase is recovered by simple separation of the phases by settling, and the nitroxide is isolated by evaporating the organic solvent under reduced pressure.

2. The process as claimed in claim 1, **characterized in that** the peracid/secondary amine molar ratio ranges from 1.5 to 2.

3. The process as claimed in claim 1 or 2, **characterized in that** the pH of the aqueous phase of the two-phase medium ranges from 5 to 9.

4. The process as claimed in any one of claims 1 to 3, **characterized in that** the temperature is between -5°C and +30°C.

5. The process as claimed in any one of claims 1 to 4, **characterized in that** the organic solvent is chosen from

aliphatic and cycloaliphatic hydrocarbons, aromatic hydrocarbons, chlorinated hydrocarbons and aliphatic acid esters.

6. The process as claimed in claim 5, **characterized in that** the aliphatic hydrocarbons are pentane, hexane or heptane, the chlorinated hydrocarbons are methylene chloride or 1,2-dichloroethane and the aliphatic acid esters are ethyl acetate or ethyl propionate.

7. The process as claimed in claim 1, **characterized in that** the aliphatic peracid is peracetic acid, perpropionic acid or perbutanoic acid.

8. The process as claimed in claim 1, **characterized in that** the aqueous basic solution is an aqueous solution of a carbonate or hydrogen carbonate of an alkali metal or of an alkaline-earth metal.

9. The process as claimed in claim 8, **characterized in that** the alkali metal carbonate or hydrogen carbonate is $NaHCO_3$, $KHCO_3$, $K_2CO_3$ or $Na_2CO_3$.

10. The process as claimed in any one of claims 1 to 9, in which the secondary amine is chosen from the compounds represented by the formulae below in which $R^a$, $R^b$, $R^c$ and $R^d$, which may be identical or different, represent a linear or branched alkyl radical containing a number of carbon atoms ranging from 1 to 10, or $R^a$ and $R^b$ form with the carbon atom which bears them, or $R^c$ and $R^d$ form with the carbon atom which bears them, a cycloalkyl containing a number of carbon atoms ranging from 3 to 10 or a spiran, cholestane or androstane residue:

- the compounds of formula (I):

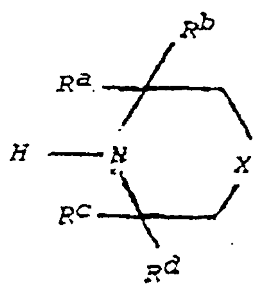

(I)

in which X represents a divalent radical chosen from the following radicals: $>CH_2$ ; $>C=O$ ; —O— ;

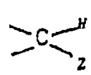

in which Z represents a monovalent residue chosen from -CN, -NHR, -OR, $-N=C(R)_2$ in which R represents a hydrogen atom, a linear or branched alkyl radical containing a number of carbon atoms ranging from 1 to 10, a benzyl radical, a phenyl radical, -CN, $-C(O)R^1$; $>C=N-R^1$, $>C(OR^1)_2$ in which $R^1$ has the same meaning as R ;
- the compounds of formula (II):

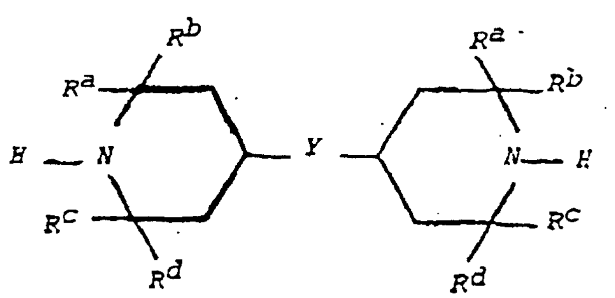

(II)

in which Y represents a divalent residue chosen.from:

-OC (O) - $(CR^2R^3)_n$-C (O) O-,

-NH- $(CR^2R^3)_n$NH-,

-NHC (O) - $(CR^2R^3)_n$-C (O) NH,

-S-, -O-, $R^2$ and $R^3$, which may be identical or different, represent a hydrogen atom or a linear or branched alkyl radical containing a number of carbon atoms ranging from 1 to 10, and n represent an integer ranging from 0 to 20;

- the compounds of formula (III):

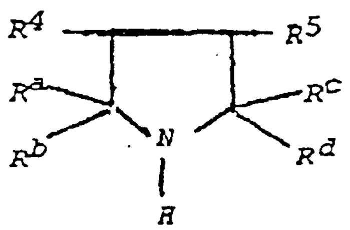

(III)

in which $R^4$ represents a hydrogen atom or a linear or branched alkyl radical containing a number of carbon atoms ranging from 1 to 10, and $R^5$ represents a hydrogen atom, $-CH_3$, $-C_2H_5$, $-CH_2OH$, $-C(O)NH_2$, -OH or -CHO;

- the compounds of formula (IV):

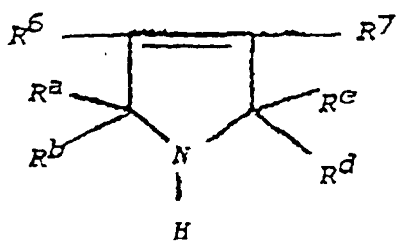

(IV)

in which $R^6$ has the same meaning as $R^4$ and $R^7$ has the same meaning as $R^5$,

- the 1,1,3,3-tetramethylpyrrolopyridines of formula (V):

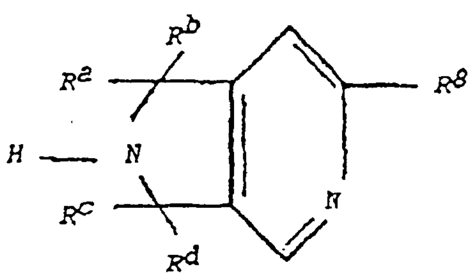

(V)

and the 1,1;3,3-tetramethyl-2,3-dihydroisoindoles of formula (VI):

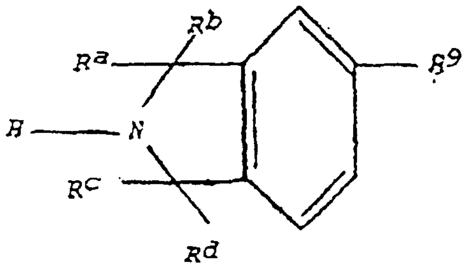

(VI)

in which $R^8$ and $R^9$ represent a hydrogen atom or a linear or branched alkyl radical containing a number of carbon atoms ranging from 1 to 10;

- the compounds of formula (VII):

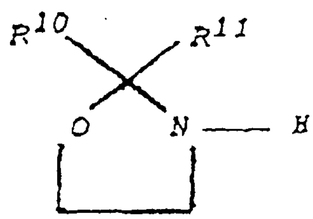

(VII)

in which $R^{10}$ and $R^{11}$, which may be identical or different, represent a linear or branched alkyl radical containing a number of carbon atoms ranging from 1 to 20, or a carboxyalkyl radical $-(CH_2)mCO_2H$ in which: m = 1 to 20;

- the compounds of formula (VIII):

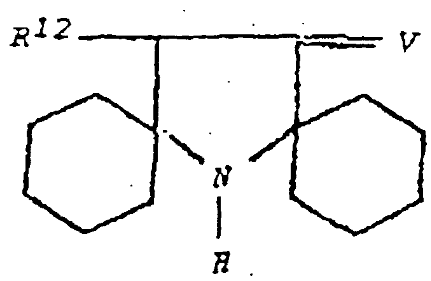

(VIII)

in which $R^{12}$ represents a hydrogen atom, a linear or branched alkyl residue containing a number of carbon atoms ranging from 1 to 20, or a $-C(O)NH_2$ radical, V=0; S, NH;

- the compounds of formula (IX):

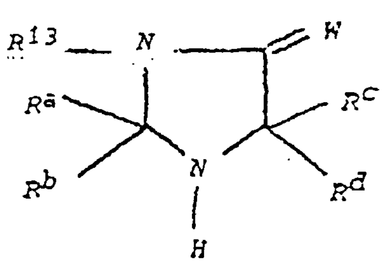

(IX)

in which $R^{13}$ has the same meaning as $R^{12}$ and W has the same meaning as V in formula (VIII):

- the compounds of formula (X):

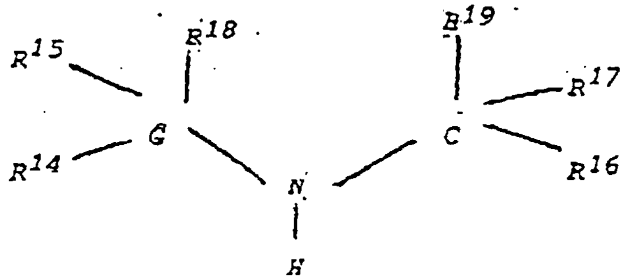

(X)

in which $R^{14}$, $R^{15}$, $R^{16}$, $R^{17}$, $R^{18}$ and $R^{19}$, which may be identical or different, represent a linear or branched alkyl radical containing a number of carbon atoms ranging from 1 to 20, a cycloalkyl radical containing at least 3 carbon atoms, a phenyl radical or a benzyl radical, or alternatively $R^{14}$ and $R^{15}$ form with the carbon atom which bears them, or alternatively $R^{16}$ and $R^{17}$ form with the carbon atom which bears them, a cycloalkyl containing a number of carbon atoms ranging from 3 to 10;

- 1,2,3,6-tetrahydro-2,2,6,6-tetramethylpyridine;
- 3,3-dimethyl-1-oxa-4-azaspiro[4.5]decane;
- 2,3,3,5,5-pentamethylmorpholine;
- 3,3,5,5-tetramethyl-2-methylenemorpholine;
- N-(2,2,6,6-tetramethylpiperidyl-4)-ε-caprolactam;
- 4,4'-dimethylspiro(5α-cholestane-3,2'-oxazolidine);
- the compounds of formula (XI):

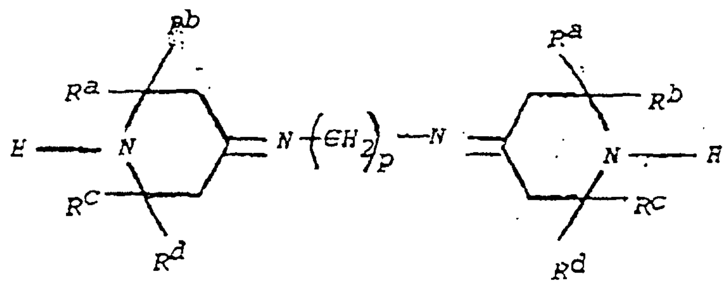

(XI)

with p = 0 to 20;

- the compounds of formula (XII):

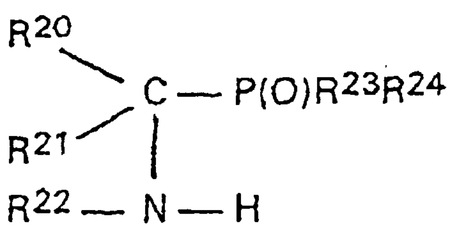

(XII)

in which in which $R^{20}$ and $R^{21}$, which may be identical or different, represent a hydrogen atom, a linear, branched or cyclic alkyl radical containing a number of carbon atoms ranging from 1 to 10, an aryl radical, or an aralkyl radical, or alternatively $R^{20}$ and $R^{21}$ are linked together so as to form a ring including the carbon atom bearing said radicals $R^{20}$ and $R^{21}$, said ring containing a number of carbon atoms, including the carbon bearing the radicals $R^{20}$ and $R^{21}$, ranging from 3 to 8; $R^{22}$ represents a linear or branched, saturated or unsaturated hydrocarbon-based radical which may comprise at least one ring, said radical containing a number of carbon atoms ranging from 1 to 30; $R^{23}$ and $R^{24}$, which may be identical or different, represent a linear or branched alkyl radical containing a number of carbon atoms ranging from 1 to 20, a cycloalkyl, aryl, alkoxy, aryloxy, aralkyloxy, perfluoroalkyl, aralkyl, dialkyl or diarylamino, alkylarylamino or thioalkyl radical, or alterna-

tively $R^{23}$ and $R^{24}$ are linked together so as to form a ring including a phosphorus atom, said heterocycle containing a number of carbon atoms ranging from 2 to 4 and also possibly containing one or more oxygen, sulfur or nitrogen atoms;

- the oxa-1-diaza-oxo-spirodecanes of formula (XIII):

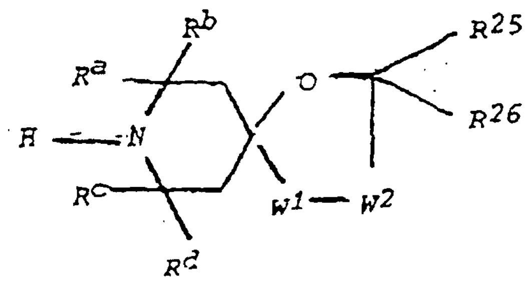

(XIII)

in which $W^1$ and $W^2$ are different and represent -C(O)- or -NH-, $R^{25}$ and $R^{26}$, which may be identical or different, represent a hydrogen atom, a linear or branched alkyl radical containing a number of carbon atoms ranging from 1 to 18, a phenyl radical, a naphthyl radical or a phenylalkyl radical, or alternatively $R^{25}$ and $R^{26}$ form, together with the carbon atom which bears them, a cycloalkyl containing a number of carbon atoms ranging from 5 to 12 or a 2,2,6,6-tetramethylpiperidyl radical;

- the secondary polyamines represented by the general formula (XIV) :

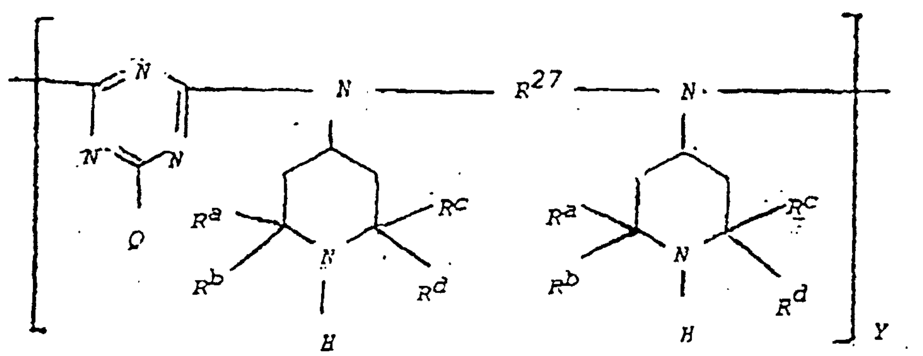

(XIV)

in which y = 1 to 20, $R^{27}$ represents an alkylene radical containing a number of carbon atoms ranging from 2 to 12 which may be interrupted with an -O- or -$NR^{28}$-, $R^{28}$ denoting a hydrogen atom, an alkyl radical containing a number of carbon atoms ranging from 1 to 12, a cycloalkyl radical, Q represents a radical -$OR^{29}$, -$NHR^{30}$ or -$NR^{30}R^{31}$ in which $R^{29}$ represents a linear or branched alkyl radical containing a number of carbon atoms ranging from 1 to 12, a $C_3$-$C_{12}$ alkoxyalkyl radical, a cyclohexyl radical, a benzyl radical, a phenyl radical, a tolyl radical or a 2,2,6,6-tetramethylpiperidyl residue, $R^{30}$ and $R^{31}$ have the same meaning as $R^{29}$, or alternatively $R^{30}$ and $R^{31}$ form, together with the nitrogen atom which bears them, a 5-, 6- or 7-membered heterocyclic radical which may also contain an oxygen atom;

- the secondary polyamine represented by formula (XV) :

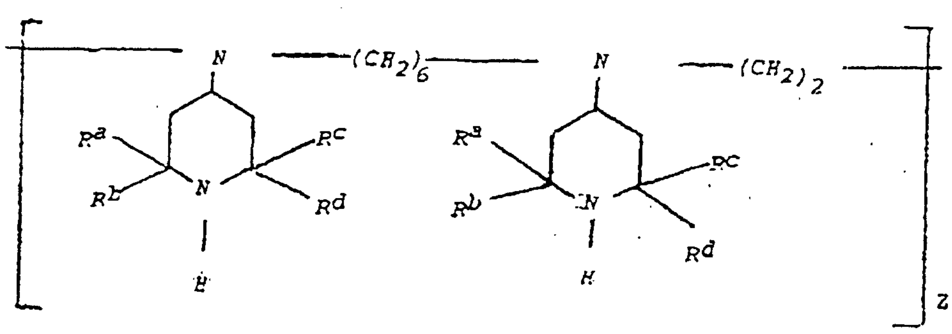

(XV)

in which z = 1 to 200.

**11.** The process as claimed in claim 10, **characterized in that** the secondary amine is:

- 2,2,6,6-tetramethylpiperidine (formula (I) in which X = $-CH_2-$ and $R^a = R^b = R^c = R^d = -CH_3$),
- 4-hydroxy-2,2,6,6-tetramethylpiperidine (formula (I) in which X = >CHZ with Z = OH and $R^a = R^b = R^c = R^d = -CH_3$),
- 4-oxo-2,2,6,6-tetramethylpiperidine (formula (I) in which X = -C(O) - and $R^a = R^b = R^c = R^d = -CH_3$),
- bis(2,2,6,6-tetramethylpiperidine) sebacate (formula (II) in which Y = $-OC(O)(CH_2)_8C(O)O$ and $R^a = R^b = R^c = R^d = -CH_3$),
- 2,2,3,4,5,5-hexamethylpyrrolidine (formula (III) in which $R^4 = R^5 = -CH_3$ and $R^a = R^b = R^c = R^d = -CH_3$),
- 2,2,4,5,5-pentamethyl-3-pyrrolidinylcarboxamide (formula (III) in which $R^4 = CH_3$, $R^5 = -C(O)NH_2$ and $R^a = R^b = R^c = R^d = -CH_3$),
- 2,2,4,5,5-pentamethyl-3-pyrrolinylcarboxamide (formula (IV) in which $R^6 = -CH_3$, $R^7 = -C(O)NH_2$ and $R^a = R^b = R^c = R^d = -CH_3$),
- diethyl 2,2-dimethyl-1-(1,1-dimethylamino) propylphosphonate ($R^{20}$ = -H, $R^{21} = R^{22} = (CH_3)_3C-$, $R^{23} = R^{24} = C_2H_5O-$),
- 2,2,7,7,9,9-hexamethyl-1-oxa-4,8-diaza-3-oxospiro[4.5]decane (formula XIII) in which $R^{25} = R^{26} = -CH_3$, W1 = -NH- and W2 = -C(O)-),
- 2,2,4,4-tetramethyl-3,20-diaza-7-oxa-21-oxo-dispiro[5.1.11.2]heneicosane sold by the company Hoechst under the name Hostavin® N-20 (formula (XIII) in which $R^{25}$ and $R^{26}$ form a $-(CH_2)_{11}-$ ring, W1 = -C(O)-, W2 = -NH- and $R^a = R^b = R^c = R^d = -CH_3$),
- the polyamine of formula (XIV) in which $R^{27} = -(CH_2)_6-$, Q = $NH\text{-}tC_8H_{17}$ and $R^a = R^b = R^c = R^d = -CH_3$, sold by the company Ciba-Geigy under the name Chimassorb® 944.